# EUROPEAN PATENT APPLICATION

(11) **EP 4 830 758 A1**
(43) Date of publication of application: **28.10.2026**
(21) Application number: 24907519.3
(22) Date of filing: 19.12.2024
(51) Int. Cl.: A61M 39/06

(54) **VALVE-EQUIPPED INDWELLING NEEDLE ASSEMBLY**

(30) Priority: 21.12.2023 JP 2023216046
(71) Applicant: Nipro Corporation, Settsu-shi, Osaka 566-8510 (JP)
(72) Inventor: SAKAMOTO, Shingo, Settsu-shi, Osaka 566-8510 (JP)
(74) Representative: Slingsby Partners LLP
(86) International application number: PCT/JP2024/045053
(87) International publication number: WO 2025/135135

(57) **Abstract**

A valved indwelling needle assembly 10 includes an inner needle 16 inserted through an outer needle 72 and an outer needle hub 74. A lumen 76 of the outer needle hub 74 houses a valve body 94 opening and closing through elastic deformation, and a plunger 118 arranged further on a proximal end side than the valve body 94 and turning the valve body 94 into a communicating state by pushing toward a distal end while receiving a returning force toward a proximal end due to a reaction force from the valve body 94 in its communicating state. An entire axial length L0 of the plunger 118 is at least three times an axial length L2 of a tapered part 124 provided to its distal end side, the plunger 118 is entirely located within the outer needle hub 76, and the axial length of the tapered part 124 is larger than an effective radius r of the valve body 94.

## Description

### TECHNICAL FIELD

This invention relates to an indwelling needle assembly in which an inner needle having an inner needle hub is inserted through an outer needle and an outer needle hub, and particularly to a valved indwelling needle assembly in which the lumen of the outer needle hub can be switched between communication and blockage by a valve body.

### BACKGROUND ART

Conventionally, an indwelling needle has been known to constitute an administration port to a blood vessel or an outlet of blood for infusion or dialysis, etc., and an indwelling needle assembly used for puncturing a blood vessel with an indwelling needle is also known. As shown in Japanese Unexamined Patent Publication No. JP-A-2011-115630 (Patent Document 1), for example, an indwelling needle assembly includes an outer needle and an outer needle hub that constitute an indwelling needle, and an inner needle that has an inner needle hub and is inserted through the outer needle and the outer needle hub. The outer needle punctures the blood vessel together with the inserted inner needle, and by pulling the inner needle out of the outer needle while maintaining the outer needle in a puncture state, the indwelling needle including the outer needle is indwelled in the blood vessel in the puncture state.

Also known is a valved indwelling needle, as shown in Patent Document 1, in which a valve body is provided in the lumen of the outer needle hub to prevent blood from leaking to the outside through the lumen of the outer needle and the outer needle hub that have been indwelled in a puncture state into a blood vessel. In the valved indwelling needle, for example, the lumen of the outer needle hub is blocked by the valve body when the inner needle is removed, and the valve body gets in communication when a male luer is connected to the outer needle hub. By inserting the inner needle through the outer needle and the outer needle hub of the valved indwelling needle, a valved indwelling needle assembly is formed.

### BACKGROUND ART DOCUMENT

### PATENT DOCUMENT

Patent Document 1: JP-A-2011-115630

### SUMMARY OF THE INVENTION

### PROBLEM THE INVENTION ATTEMPTS TO SOLVE

In Patent Document 1, a tubular plunger is housed in the lumen of the outer needle hub on the proximal end side of the valve body. The plunger is pushed to the distal end side and penetrates a slit in the valve body, thereby shifting the valve body from the blocking state to the communicating state. The plunger is pushed to the distal end side, for example, when a male luer is inserted into the proximal end opening of the outer needle hub and the distal end of the male luer comes into contact with the plunger.

In some valved indwelling needles, when the male luer is removed from the outer needle hub and the pushing force toward the distal end side against the plunger is released, the plunger is pushed back to the proximal end side by the elasticity of the valve body, and the valve body is restored to the blocking state. In this case, the distal end portion of the plunger is tapered to effectively exert the force of the elasticity of the valve body to push the plunger back to the proximal end side. The valve body that has been deformed to the communicating state is pressed against the tapered part, and when the pushing force to the distal end side against the plunger is released, the plunger is moved to the proximal end side by the returning force to the proximal end side based on the elasticity of the valve body, so that the valve body is restored to the blocking state. Therefore, when the proximal end opening of the outer needle hub is opened, the lumen of the outer needle hub is blocked by the valve body, preventing blood leakage.

However, the examination by the inventor revealed that the returning force based on the elasticity of the valve body acting on the plunger varies depending on slight differences in the total length, hardness, thickness, etc. of the standardized male luer inserted into the outer needle hub, and in some cases, the plunger is not pushed back sufficiently and the valve body may not achieve a proper blocking state. Specifically, for example, it was confirmed that when the male luer is thick and hard, the amount of pushing of the male luer to the distal end side relative to the outer needle hub is insufficient, and even when the male luer is removed from the outer needle hub, the plunger may not be pushed back sufficiently and the valve body may not achieve the proper blocking state.

It was also confirmed that the insertion length of the male luer inserted from the proximal end side into the outer needle hub varies due to variations in manufacturing errors and hardness, and differences in the user's pushing force, making it difficult to obtain a stable returning force to push back the plunger.

Besides, in some cases, a protector protecting a needle tip of the inner needle pulled out from the outer needle is arranged between leg-shaped parts provided on the proximal end side of the plunger. In such cases, there is a possibility that a displacement restricting structure that restricts the displacement of the plunger to the proximal end side with respect to the outer needle hub may interfere with the protector.

It was further observed that as the outer needle hub is downsized, the valve body may be pinched between the outer needle hub and the plunger, thereby hindering the plunger from moving to the distal end side.

It is therefore objects of the present invention to solve any of the aforementioned problems. It is one object of the present invention to provide a valved indwelling needle assembly of novel structure which is able to improve the stability of switching the valve body from the communicating state to the blocking state by pushing the plunger back to the proximal end side based on the elasticity of the valve body.

It is another object of the present invention to provide a valved indwelling needle assembly of novel structure which is able to reduce the situation in which the displacement restricting structure that restricts the movement of the plunger interferes with the protector.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a valved indwelling needle assembly comprising: an inner needle including an inner needle hub, the inner needle being inserted through an outer needle and an outer needle hub; a valve body opening and closing through elastic deformation; and a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein the plunger includes a tapered part on the distal end side, an entire axial length of the plunger is at least three times as large as an axial length of the tapered part, the plunger is entirely located within the outer needle hub, and the axial length of the tapered part is larger than an effective radius of the valve body where free deformation is allowed on a distal end surface of the valve body.

According to the valved indwelling needle assembly structured following the present preferred embodiment, the axial length of the tapered part is larger than the effective radius of the valve body, which enables the valve body to open and close smoothly. Moreover, it is possible to stably obtain the returning force exerted on the tapered part of the plunger due to the elastic reaction force from the valve body. The entire axial length of the plunger is at least three times as large as the axial length of the tapered part, which is set sufficiently long. This makes it easier to place the proximal end of the plunger closer to the proximal end opening of the outer needle hub in comparison with the conventional structure. This allows the plunger to move a longer distance relative to the valve body when inserting the male luer into the outer needle hub. By so doing, even when the axial length of the male luer is short or when the insertion of the male luer into the outer needle hub is shallow due to insufficient pushing or the like, for example, it is easy to reliably obtain a large reaction force (returning force) of the valve body acting on the tapered part of the plunger, thereby improving the stability of the blocking function of the valve body.

A second preferred embodiment provides the valved indwelling needle assembly according to the first preferred embodiment, wherein the axial length of the tapered part is at least 3 mm, and in a pushing region of the plunger that extends 3 mm further from an open and communicating location where an opening of the valve body opened by the plunger being pushed into the valve body reaches a size equal to an inner hole diameter of the plunger, a value of resistance to pushing the plunger into the valve body is kept greater than that at the open and communicating location.

According to the second preferred embodiment, the pushing region in which the plunger that has reached the open and communicating location can move further to the distal end side is provided, and the tapered part has a taper length that allows the elastic returning force of the valve body to act sufficiently on the tapered part even in the pushing region. With these configurations, it is possible to set a wider range of the length of compatibility of the male luer inserted from the proximal end side into the outer needle hub (the axial length of the male luer for which the plunger can be pushed to the location where the effective returning force is applied) than that in the conventional structure. Therefore, even if the axial insertion length of the male luer varies due to variations in manufacturing error or hardness, differences in the pushing force by the user, and other factors, it is possible to stably obtain the returning force to the proximal end side for the plunger that is pushed into the valve body.

A third preferred embodiment provides the valved indwelling needle assembly according to the first or second preferred embodiment, wherein the axial length of the tapered part is at least twice as large as an outer diameter of a distal end part of the tapered part, while being at least twice as large as the effective radius of the valve body, and the outer diameter of the distal end part of the tapered part is equal to or less than the effective radius of the valve body where free deformation is allowed on the distal end surface of the valve body.

According to the third preferred embodiment, the axial length of the tapered part of the plunger is at least twice as large as the outer diameter of the distal end part, and the outer diameter of the distal end part of the tapered part is equal to or less than the effective radius of the valve body. Since the distal end of the plunger is thin, it is easy to reliably obtain a large inclination angle of the tapered part. Besides, since the axial length of the tapered part is long, the range of the axial length of the male luer for which the plunger can be returned to the initial position by the elasticity of the valve body can be wider than that in the conventional structure. Therefore, even if there are variations in dimension and hardness of the male luer and the outer needle hub due to manufacturing errors or other factors, or variations in amount of pushing of the male luer into the outer needle hub, it is possible to stably obtain the returning force for the plunger that is pushed into the valve body based on the elasticity of the valve body.

A fourth preferred embodiment provides the valved indwelling needle assembly according to any one of the first through third preferred embodiments, wherein the plunger comprises a tubular part whose distal end side has a tapered shape, and a leg-shaped part including a plurality of legs extending toward the proximal end side, the tubular part includes a restricting step that restricts a movement of the plunger to the proximal end side relative to the outer needle hub by contact with an outer needle hub side, a protector is arranged in the leg-shaped part, the protector protecting a needle tip when the inner needle is withdrawn, and an axial length of the leg-shaped part is longer than an axial length of the tubular part.

According to the fourth preferred embodiment, since the plunger includes a leg-shaped part having a plurality of legs extending from the tubular part to the proximal end side, a space for disposing the protector can be reliably obtained between those plurality of legs. For example, by locating the portion of the protector, which is enlarged in diameter dimension, between the circumferential interval of the plurality of legs, it is easier to avoid increase in diameter of the outer needle hub than in the case where the protector is surrounded by the tubular plunger about the entire circumference.

The axial length of the leg-shaped part of the plunger is lengthened, and the restricting step is formed in the tubular part of the plunger. Accordingly, the space for restricting displacement of the plunger to the proximal end side and the space for disposing the protector can be separated in the outer needle hub. Therefore, it is possible to prevent the displacement restricting structure that restricts the displacement of the plunger to the proximal end side from interfering with the protector.

A fifth preferred embodiment provides the valved indwelling needle assembly according to any one of the first through fourth preferred embodiments, wherein the lumen of the outer needle hub includes a deformation allowance part extending straightly from a distal end of the valve body toward the distal end side with a substantially constant diameter while having an inner diameter larger than a maximum diameter of the tapered part, an axial length of the deformation allowance part is larger than the effective radius of the valve body, and the axial length of the tapered part is longer than an axial distance from a proximal end of the outer needle hub to a proximal end of the plunger that is located on the distal end side with respect to the proximal end of the outer needle hub.

According to the fifth preferred embodiment, the axial distance from the proximal end of the outer needle hub to the proximal end of the plunger is comparatively short. Thus, for example, even when the insertion length of the male luer into the outer needle hub is short, the plunger is pushed sufficiently to the distal end side, and the valve body is turned into the communicating state with stability. Meanwhile, by providing the deformation allowance part, the valve body is less likely to be clasped between the outer needle hub and the plunger when the plunger is pushed to the distal end side. This reduces the situation in which the clasped valve body makes it difficult to push the plunger to a predetermined location.

A sixth preferred embodiment provides a valved indwelling needle assembly comprising: an inner needle including an inner needle hub, the inner needle being inserted through an outer needle and an outer needle hub; a valve body opening and closing through elastic deformation; and a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein the plunger includes a tapered part on the distal end side, an axial length of the tapered part is at least 3 mm, and in a pushing region of the plunger that extends 3 mm further from an open and communicating location where an opening of the valve body opened by the plunger being pushed into the valve body reaches a size equal to an inner hole diameter of the plunger, a value of resistance to pushing the plunger into the valve body is kept greater than that at the open and communicating location.

According to the valved indwelling needle assembly structured following the present preferred embodiment, the pushing region in which the plunger that has reached the open and communicating location can move further to the distal end side is provided, and the tapered part has a taper length that allows the elastic returning force of the valve body to act sufficiently on the tapered part even in the pushing region. With these configurations, it is possible to set a wider range of the length of compatibility of the male luer inserted from the proximal end side into the outer needle hub (the axial length of the male luer for which the plunger can be pushed to the location where the effective returning force is applied) than that in the conventional structure. Therefore, even if the axial insertion length of the male luer varies due to variations in manufacturing error or hardness, differences in the pushing force by the user, and other factors, it is possible to stably obtain the returning force to the proximal end side for the plunger that is pushed into the valve body.

A seventh preferred embodiment provides a valved indwelling needle assembly comprising: an inner needle including an inner needle hub, the inner needle being inserted through an outer needle and an outer needle hub; a valve body opening and closing through elastic deformation; and a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein the plunger includes a tapered part on the distal end side, an axial length of the tapered part is at least twice as large as an outer diameter of a distal end part of the tapered part, while being at least twice as large as an effective radius of the valve body, and the outer diameter of the distal end part of the tapered part is equal to or less than the effective radius of the valve body where free deformation is allowed on a distal end surface of the valve body.

According to the valved indwelling needle assembly structured following the present preferred embodiment, the axial length of the tapered part of the plunger is at least twice as large as the outer diameter of the distal end part, while being at least twice as large as an effective radius of the valve body, and the outer diameter of the distal end part of the tapered part is equal to or less than the effective radius of the valve body. The axial length of the tapered part of the plunger is longer than the effective radius of the valve body, which makes it easy to avoid increase in diameter of the outer needle hub. Besides, since the distal end of the plunger is thin, it is easy to reliably obtain a large inclination angle of the tapered part. Besides, since the axial length of the tapered part is long, the range of the axial length of the male luer for which the plunger can be returned to the initial position by the elasticity of the valve body can be wider than that in the conventional structure. Therefore, even if there are variations in dimension and hardness of the male luer and the outer needle hub due to manufacturing errors or other factors, or variations in amount of pushing of the male luer into the outer needle hub, it is possible to stably obtain the returning force for the plunger that is pushed into the valve body based on the elasticity of the valve body. The valved indwelling needle assembly in the present preferred embodiment is particularly suitable for infusion.

An eighth preferred embodiment provides a valved indwelling needle assembly comprising: an inner needle including an inner needle hub, the inner needle being inserted through an outer needle and an outer needle hub; a valve body opening and closing through elastic deformation; and a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein the plunger comprises a tubular part whose distal end side has a tapered shape, and a leg-shaped part including a plurality of legs extending toward the proximal end side, the tubular part includes a restricting step that restricts a movement of the plunger to the proximal end side relative to the outer needle hub by contact with an outer needle hub side, a protector is arranged in the leg-shaped part, the protector protecting a needle tip when the inner needle is withdrawn, and an axial length of the leg-shaped part is longer than an axial length of the tubular part.

According to the valved indwelling needle assembly structured following the present preferred embodiment, since the plunger includes a leg-shaped part having a plurality of legs extending from the tubular part to the proximal end side, a space for disposing the protector can be reliably obtained between those plurality of legs. For example, by locating the portion of the protector, which is enlarged in diameter dimension, between the circumferential interval of the plurality of legs, it is easier to avoid increase in diameter of the outer needle hub than in the case where the protector is surrounded by the tubular plunger about the entire circumference.

The axial length of the leg-shaped part of the plunger is lengthened, and the restricting step is formed in the tubular part of the plunger. Accordingly, the space for restricting displacement of the plunger to the proximal end side and the space for disposing the protector can be separated in the outer needle hub. Therefore, it is possible to prevent the displacement restricting structure that restricts the displacement of the plunger to the proximal end side from interfering with the protector.

A ninth preferred embodiment provides a valved indwelling needle assembly comprising: an inner needle including an inner needle hub, the inner needle being inserted through an outer needle and an outer needle hub; a valve body opening and closing through elastic deformation; and a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein the plunger includes a tapered part on the distal end side, and the lumen of the outer needle hub includes a deformation allowance part extending straightly from a distal end of the valve body toward the distal end side with a substantially constant diameter while having an inner diameter larger than a maximum diameter of the tapered part, an axial length of the deformation allowance part is larger than an effective radius of the valve body, and an axial length of the tapered part is longer than an axial distance from a proximal end of the outer needle hub to a proximal end of the plunger that is located on the distal end side with respect to the proximal end of the outer needle hub.

According to the valved indwelling needle assembly structured following the present preferred embodiment, the axial distance from the proximal end of the outer needle hub to the proximal end of the plunger is comparatively short. Thus, for example, even when the insertion length of the male luer into the outer needle hub is short, the plunger is pushed sufficiently to the distal end side, and the valve body is turned into the communicating state with stability. Meanwhile, by providing the deformation allowance part, the valve body is less likely to be clasped between the outer needle hub and the plunger when the plunger is pushed to the distal end side. This reduces the situation in which the clasped valve body makes it difficult to push the plunger to a predetermined location.

A tenth preferred embodiment provides a valved indwelling needle assembly comprising: an inner needle including an inner needle hub, the inner needle being inserted through an outer needle and an outer needle hub; a valve body opening and closing through elastic deformation; and a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein the plunger includes a tapered part on the distal end side, the lumen of the outer needle hub includes a constricted part and a caulking pin that decreases in diameter toward the distal end side and is fastened by caulking to an inner circumferential surface of the constricted part, the caulking pin fixing the outer needle, when the plunger is pushed into the valve body, the plunger moves to a location where the tapered part enters a radial inside of the caulking pin, and in a region from a distal end of the valve body to the caulking pin, the lumen of the outer needle hub includes a deformation allowance part that has a diameter larger than a maximum diameter of the tapered part and extends straightly longer than an effective radius of the valve body.

According to the valved indwelling needle assembly structured following the present preferred embodiment, even if the constricted part is provided in the lumen of the outer needle hub to fasten the caulking pin by caulking, the straight part is set between the valve body and the constricted part, so that the valve body, which has been deformed by the pushing of the plunger, is less likely to reach the constricted part to be inserted into the radial inside of the caulking pin. Therefore, the valve body is prevented from being clasped between the caulking pin having a tapered shape corresponding to the constricted part and the plunger, thereby stably allowing the movement of the plunger to the distal end side. Such a configuration is preferably employed, for example, in combination with any one of the first through ninth preferred embodiments.

An eleventh preferred embodiment provides a valved indwelling needle assembly comprising: an inner needle including an inner needle hub, the inner needle being inserted through an outer needle and an outer needle hub; a valve body opening and closing through elastic deformation; and a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein the lumen of the outer needle hub houses the plunger arranged on the proximal end side with respect to the valve body and turning the valve body into the communicating state by being pushed to the distal end side, the plunger includes a tapered part on the distal end side, the valve body includes a locking groove opening on a proximal end surface into which a distal end claw part of a pusher guide that guides an axial movement of the plunger is inserted and locked, the locking groove includes an outer circumferential lightening part extending further radially inward than the distal end claw part of the pusher guide, and a radial width dimension of the outer circumferential lightening part of the locking groove is 2/3 or less than an outer diameter of a distal end part of the tapered part of the plunger.

According to the valved indwelling needle assembly structured following the present preferred embodiment, the locking groove opening on the proximal end surface of the valve body includes the outer circumferential lightening part that extends further radially inward than the distal end claw part of the pusher guide. With this configuration, the portion of the valve body that is thin-walled due to forming of the outer circumferential lightening part is not restrained by the distal end claw part of the pusher guide, but is allowed to deform. Therefore, the outer circumferential lightening part allows the valve body to be more smoothly deformed by pushing of the plunger, thereby achieving stabilization in switching the valve body to the communicating state.

The width dimension of the outer circumferential lightening part is 2/3 or less than the outer diameter of the distal end part of the tapered part, and the width dimension of the outer circumferential lightening part is not excessively large. Therefore, even if the valve body is thin-walled at the outer circumferential lightening part, the shape recovering force of the deformed valve body to its pre-deformed state is effectively exhibited, and the return of the plunger to the proximal end side based on the said shape recovering force is stably realized. Such a configuration is preferably employed, for example, in combination with any one of the first through tenth preferred embodiments.

### EFFECT OF THE INVENTION

According to any one of the first through seventh or ninth through eleventh embodiments of the present invention, in the valved indwelling needle assembly, it is possible to improve the stability of pushing the plunger back to the proximal end side based on the elasticity of the valve body.

According to the eighth preferred embodiment of the present invention, it is possible to reduce the situation in which the displacement restricting structure that restricts the displacement of the plunger to the proximal end side with respect to the outer needle hub interferes with the protector.

### BRIEF DESCRIPTION OF THE DRAWINGS

[FIG. 1] is a perspective view showing a valved indwelling needle assembly according to a first practical embodiment of the present invention.
[FIG. 2] is a cross sectional view of the valved indwelling needle assembly shown in FIG. 1, corresponding to a cross section taken along line 2-2 of FIG. 3.
[FIG. 3] is a cross sectional view taken along line 3-3 of FIG. 2.
[FIG. 4] is a perspective view of a valve body constituting the valved indwelling needle assembly shown in FIG. 1.
[FIG. 5] is a cross sectional view of the valve body shown in FIG. 4.
[FIG. 6] is a perspective view of a pusher guide constituting the valved indwelling needle assembly shown in FIG. 1.
[FIG. 7] is a perspective view showing the pusher guide of FIG. 6 from another angle.
[FIG. 8] is a perspective view of a plunger constituting the valved indwelling needle assembly shown in FIG. 1.
[FIG. 9] is a perspective view showing the plunger of FIG. 8 from another angle.
[FIG. 10] is a left side view of the plunger shown in FIG. 8.
[FIG. 11] is a cross sectional view of the plunger shown in FIG. 8, corresponding to a cross section taken along line 11-11 of FIG. 10.
[FIG. 12] is a perspective view of a protector constituting the valved indwelling needle assembly shown in FIG. 1.
[FIG. 13] is a perspective view showing the protector of FIG. 12 from another angle.
[FIG. 14] is a cross sectional view of a principal part showing a state in which a connector is connected to an outer needle unit constituting the valved indwelling needle assembly shown in FIG. 1.
[FIG. 15] is an enlarged view of a cross section taken along line 15-15 of FIG. 14.
[FIG. 16] is a graph showing measurement results of resistance to pushing acting on the plunger.

### EMBODIMENTS FOR CARRYING OUT THE INVENTION

Hereinafter, a practical embodiment of the present invention will be described with reference to the drawings.

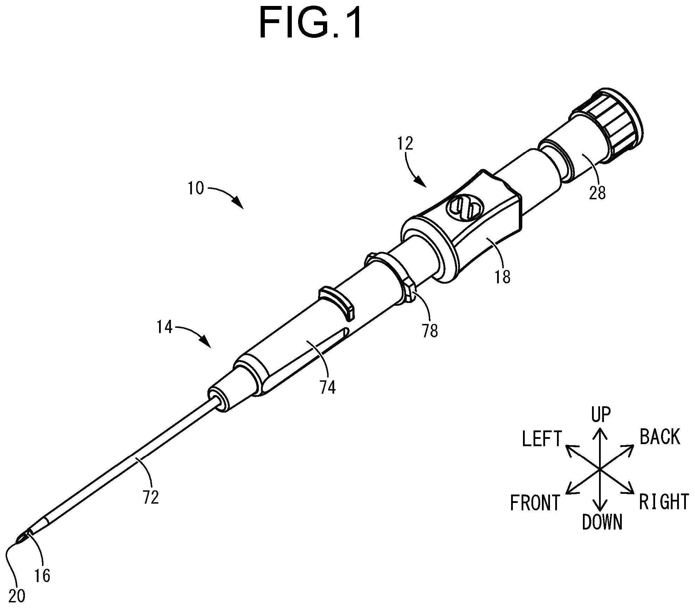

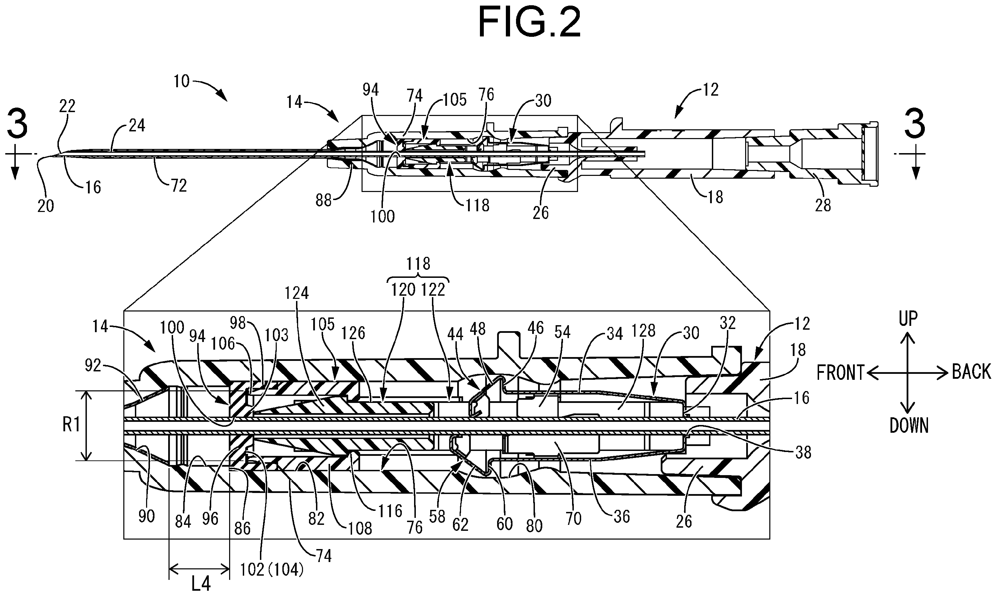

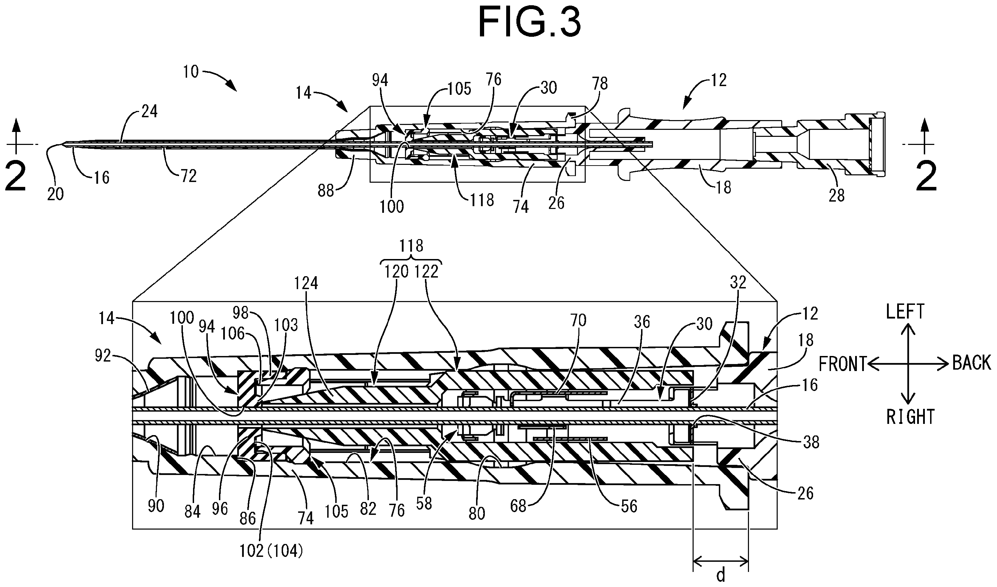

FIGS. 1 to 3 show a valved indwelling needle assembly 10 according to a first practical embodiment of the present invention. The valved indwelling needle assembly 10 is constituted by an inner needle unit 12 and an outer needle unit 14 being combined. In the following description, as a general rule, the front-back direction refers to the left-right direction in FIG. 2, which is the needle axis direction. Besides, the vertical direction refers to the vertical direction in FIG. 2, and the left-right direction refers to the vertical direction in FIG. 3. In the following explanation, as a general rule, the left side in FIG. 2, which is a needle tip 20 side (described below), is defined as the distal end, and the right side in FIG. 2 is defined as the proximal end.

The inner needle unit 12 has a structure in which an inner needle hub 18 is attached to the proximal end portion of an inner needle 16. The inner needle 16 is a hollow needle formed of medical metal such as stainless steel, and has a sharp needle tip 20 at the distal end. The needle tip 20 is formed by the distal end portion of the inner needle 16 having a blade surface 22 that extends so as to incline with respect to the needle axis. The inner needle 16 has an outer diameter dimension that is partially enlarged near the distal end portion of the inner needle 16. The proximal end portion of the inner needle 16 is inserted into the distal end portion of a tubular inner needle hub 18, and those inner needle 16 and inner needle hub 18 are fixed together by means of adhesion or other means.

The inner needle hub 18 is a member having an approximately round tubular shape, and is formed of rigid synthetic resin. The distal end part of the inner needle hub 18 includes a cylindrical connecting part 26 that can be fitted into the proximal end opening of an outer needle hub 74 described below. A filter cap 28 is fitted into the proximal end opening of the inner needle hub 18.

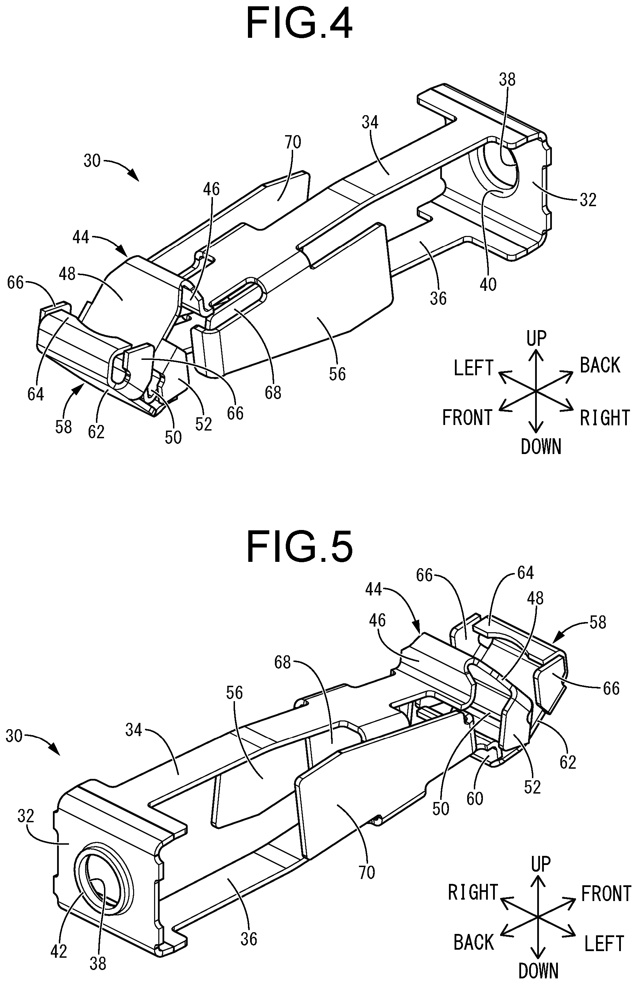

A needle tip protector 30 is attached to the inner needle 16. The needle tip protector 30 is externally attached to the inner needle 16, and moves to the distal end side of the inner needle 16 to prevent exposure of the needle tip 20 when the inner needle 16 is removed as described later. The needle tip protector 30 is made of medical metal such as stainless steel, for example. As shown in FIGS. 4 and 5, the needle tip protector 30 has a structure in which a first side plate 34 and a second side plate 36 extend out toward the needle tip 20 side from the upper and lower both edges of a bottom part 32 located on the needle base side (the opposite side of the needle tip 20) of the inner needle 16. In the present practical embodiment, the parts described later that constitute the needle tip protector 30 are integrally formed.

The bottom part 32 is an approximately rectangular flat plate. At a pair of upper and lower edges of the bottom part 32, the first side plate 34 and the second side plate 36 are integrally formed and extend out to the distal end side, which is one side in the plate thickness direction of the bottom part 32. The first side plate 34 and the second side plate 36 of the present practical embodiment are spaced apart from and face each other, and do not extend in an intersecting manner.

The bottom part 32 is penetrated by a needle insertion hole 38 in the plate thickness direction. The needle insertion hole 38 in the present practical embodiment is a circular hole that is sized such that the inner needle 16 can be inserted while a detent part 24 of the inner needle 16 does not pass through but is locked to the needle insertion hole 38. A needle guide surface 40 is provided to the opening peripheral edge on the distal end side of the needle insertion hole 38 of the bottom part 32. The needle guide surface 40 is a tapered surface that expands toward the distal end side. A reinforcing tube part 42 is provided to the opening peripheral edge on the proximal end side of the needle insertion hole 38 of the bottom part 32. The reinforcing tube part 42 has an approximately round tubular shape provided along the opening peripheral edge of the needle insertion hole 38, and protrudes from the bottom part 32 to the proximal end side.

The first side plate 34 has a band plate shape extending in the front-back direction. The proximal end portion of the first side plate 34, which is connected to the bottom part 32, has approximately the same width dimension as the bottom part 32, and the distal end portion of the first side plate 34, which is connected to a first needle-tip protection part 44 described below, is narrower than the bottom part 32.

The first needle-tip protection part 44 is provided on the distal end side of the first side plate 34. The first needle-tip protection part 44 includes a first stepped part 46 protruding from the distal end of the first side plate 34 toward the opposite side of the second side plate 36, and a first needle-tip cover part 48 protruding from the protruding distal end of the first stepped part 46 toward the second side plate 36 side while inclining toward the distal end side. A first needle-tip stopper part 50 protruding to the proximal end side is provided at the distal end portion of the first needle-tip cover part 48. A pair of first lateral guarding pieces 52, 52 that extend approximately orthogonally to the width direction are provided on widthwise both sides of the first needle-tip cover part 48.

A first lateral cover piece 54 is provided at one end edge of the first side plate 34 in the width direction. The first lateral cover piece 54 protrudes vertically inward (downward) from the first side plate 34 toward the second side plate 36. Besides, a first protrusion piece 56 is provided at the other end edge of the first side plate 34 in the width direction. The first protrusion piece 56 protrudes vertically inward (downward) toward the second side plate 36, and is spaced apart from and faces the first lateral cover piece 54 in the width direction.

The second side plate 36 has a band plate shape extending in the front-back direction. The proximal end portion of the second side plate 36, which is connected to the bottom part 32, has approximately the same width dimension as the bottom part 32, and the distal end portion of the second side plate 36, which is connected to a second needle-tip protection part 58 described below, is narrower than the bottom part 32.

The second needle-tip protection part 58 is provided on the distal end side of the second side plate 36. The second needle-tip protection part 58 includes a second stepped part 60 protruding from the distal end of the second side plate 36 toward the opposite side of the first side plate 34, and a second needle-tip cover part 62 protruding from the protruding distal end of the second stepped part 60 toward the first side plate 34 while inclining toward the distal end side. A second needle-tip stopper part 64 protruding toward the proximal end side is provided at the distal end portion of the second needle-tip cover part 62. A pair of second lateral guarding pieces 66, 66 that extend approximately orthogonally to the width direction are provided on widthwise both sides of the second needle-tip cover part 62.

A second lateral cover piece 68 is provided at the other end edge of the second side plate 36 in the width direction. The second lateral cover piece 68 protrudes vertically inward (upward) from the second side plate 36 toward the first side plate 34. Besides, a second protrusion piece 70 is provided at the one edge of the second side plate 36 in the width direction. The second protrusion piece 70 protrudes vertically inward (upward) from the second side plate 36 toward the first side plate 34, and is spaced apart from and faces the second lateral cover piece 68 in the width direction.

The protector 30 of the above construction is attached to the inner needle 16, as shown in FIGS. 2 and 3. That is, the inner needle 16 is inserted through the needle insertion hole 38 formed in the bottom part 32 of the protector 30, and is also inserted between the first needle-tip cover part 48 and the second needle-tip cover part 62. With this configuration, the inner needle 16 is arranged to penetrate between the facing surfaces of the first side plate 34 and the second side plate 36 of the protector 30, and the protector 30 is externally attached onto the inner needle 16. The inner needle 16 is located between the facing surfaces of the first lateral cover piece 54 and the second lateral cover piece 68 of the protector 30, and is surrounded in part in the needle axis direction by the first and second side plates 34, 36, the first and second lateral cover pieces 54, 68, and the first and second protrusion pieces 56, 70.

The outer needle unit 14 has a structure in which an outer needle hub 74 is attached to the proximal end portion of an outer needle 72. The outer needle 72 is a tube-shaped member made of synthetic resin or the like. The outer diameter dimension of the distal end portion of the outer needle 72 decreases toward the distal end.

The outer needle hub 74 is formed of rigid synthetic resin, for example. The outer needle hub 74 is a member having an approximately round tubular shape overall, and is penetrated by a lumen 76 in the axial direction. By fitting a connector 130 such as a syringe tip (described below) into the lumen 76 of the outer needle hub 74 from the proximal end opening, an external circuit such as a syringe or infusion line can be connected to the lumen 76 of the outer needle hub 74. In addition, a screw part 78 protruding radially outward is formed at the proximal end part of the outer needle hub 74. By the screw part 78 being screwed with a locking part (not shown) provided to the connector 130, it is possible to prevent the connector 130 from accidentally slipping out of the outer needle hub 74.

The lumen 76 of the outer needle hub 74 has a luer taper, which increases in diameter toward the proximal end side, at the proximal end part where the connector 130 described below is inserted. The connector 130 having a luer taper on its outer circumferential surface is fitted into the luer taper, so that the connector 130 and the outer needle hub 74 are positioned in close contact to each other, and liquid leakage is prevented. In the lumen 76 of the outer needle hub 74, the distal end side with respect to the proximal end part where the luer taper is set comprises a protector housing part 80. The protector housing part 80 houses the first and second needle-tip protection parts 44, 58 of the protector 30, and has a curved cross-sectional shape with a maximum inner diameter in the middle of the axial direction when viewed in vertical cross section shown in FIG. 2. In the lumen 76 of the outer needle hub 74, the distal end side with respect to the protector housing part 80 comprises a valve housing part 82 that extends in the axial direction with an approximately constant inner diameter dimension. Besides, in the lumen 76 of the outer needle hub 74, the distal end side with respect to the valve housing part 82 comprises a straight part 84 serving as a deformation allowance part that extends straightly in the axial direction with an approximately constant inner diameter dimension. The straight part 84 has a smaller diameter than the valve housing part 82, and an annular step 86 is formed at the connected portion (boundary portion) between the valve housing part 82 and the straight part 84 on the inner circumferential surface of the outer needle hub 74.

In the lumen 76 of the outer needle hub 74, the distal end side with respect to the straight part 84 comprises a caulking part 88 that decreases in diameter toward the distal end. In the caulking part 88, the proximal end portion comprises a constricted part 90 that decreases in diameter more radically than the distal end portion does. The proximal end portion of the outer needle 72 is inserted into the caulking part 88 in the lumen 76 of the outer needle hub 74, and the outer needle 72 and the outer needle hub 74 are connected by a caulking pin 92 that is fitted into the caulking part 88 and the proximal end portion of the outer needle 72. The caulking pin 92 is approximately funnel-shaped overall and is made of metal, such as stainless steel, for example. Regarding the caulking pin 92, the proximal end portion is fitted into the constricted part 90, while the distal end portion with a smaller diameter protrudes to the distal end side with respect to the constricted part 90 to be fitted into the distal end portion of the caulking part 88. The straight part 84 is provided in the outer needle unit 14 with a valve body 94, which is described below, in the region from the distal end of the valve body 94 to the caulking pin 92.

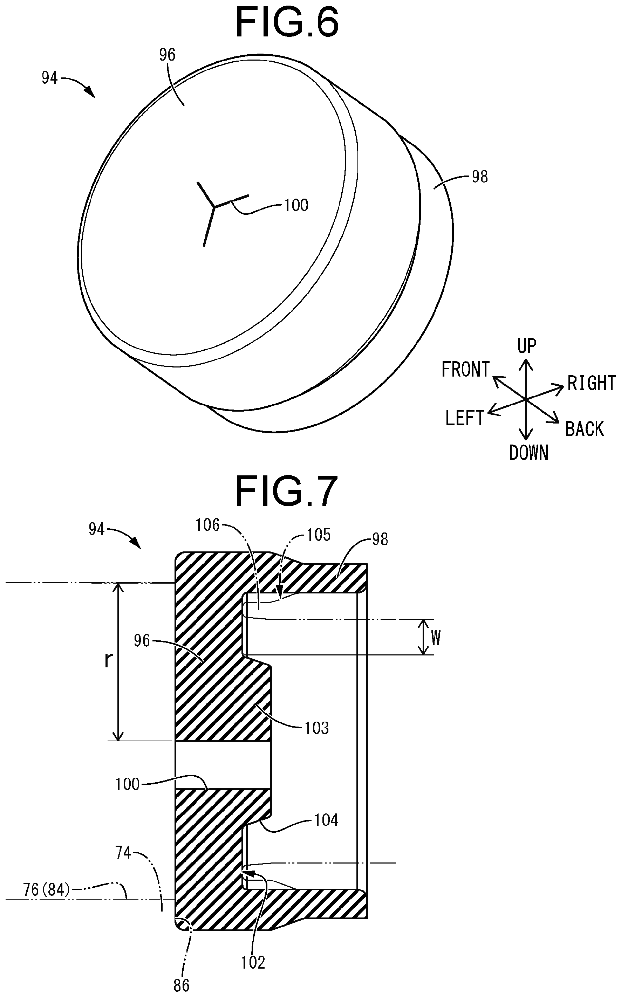

The valve body 94 is housed in the lumen 76 of the outer needle hub 74. The valve body 94 is an elastic body made of rubber, resin elastomer, or the like. As shown in FIGS. 6 and 7, the valve body 94 integrally comprises a generally circular disk-shaped valve body part 96 and a tubular support tube part 98 protruding from the outer peripheral end of the valve body part 96 to the proximal end side. In the center of the valve body part 96, a slit 100 is formed so as to pass through the valve body part 96 in the axial direction. The slit 100 extends radially outward from the diametrical center of the valve body part 96, and in the present practical embodiment, the slit 100 extends in three mutually different radial directions. The valve body 94 opens and closes the slit 100 through elastic deformation, thereby switching the distal end side and the proximal end side of the valve body part 96 between the communicating state and the blocked state. The slit 100 may have a straight-line shape, or a radial shape extending in four or more mutually different radial directions.

The valve body part 96 includes a locking groove 102 opening on the proximal end surface. The locking groove 102 is an annular concave groove extending in the circumferential direction around the outer peripheral portion of the valve body part 96 along the inner circumference of the support tube part 98. The valve body part 96 is axially thin-walled at the portion forming the locking groove 102. Regarding the valve body part 96, the radially inner side with respect to the locking groove 102 comprises a thick convex part 103 that is thicker than the portion forming the locking groove 102 and protrudes to the proximal end side. Besides, regarding the locking groove 102, the radially inner portion with respect to a distal end claw part 106 of a pusher guide 105, which will be described later, comprises an outer circumferential lightening part 104. The outer circumferential lightening part 104 constitutes a part of the locking groove 102, and is annularly provided around the thick convex part 103. The thick convex part 103 and the outer circumferential lightening part 104 may be omitted from the valve body part 96 so that the proximal end surface of the valve body part 96 is flat.

The valve body 94 is inserted into the valve housing part 82 of the outer needle hub 74 from the proximal end side. The outer circumferential surface of the valve body part 96 is pressed against the inner circumferential surface of the outer needle hub 74 in the valve housing part 82, and the valve body part 96 is fitted in the outer needle hub 74 in a state of being compressed radially inward. Besides, the outer peripheral end of the distal end surface of the valve body part 96 is overlapped on the step 86 of the inner circumferential surface of the outer needle hub 74, and the valve body 94 is axially positioned with respect to the outer needle hub 74. The outer diameter of the valve body part 96 is larger than the inner diameter of the outer needle hub 74 in which the valve body part 96 is located, and is attached to the outer needle hub 74 in a radially compressed state. With this configuration, the valve body 94 readily generates a returning force against the pusher guide 105 when the pusher guide 105 moves to the distal end side.

The axial length L4 of the straight part 84 (see FIG. 2) is suitably longer than the effective radius r of the valve body 94 (see FIG. 7), and more suitably at least 1.5 times as large as the effective radius r of the valve body 94. The axial length L4 of the straight part 84 is suitably 2.5 times or less as large as the effective radius r of the valve body 94, and more suitably twice or less as large as the effective radius r of the valve body 94, which prevents the outer needle hub 74 from being excessively long. The effective radius r of the valve body 94 refers to the radius of the portion on the distal end surface of the valve body 94 where free deformation is allowed when a plunger 118, described below, is pushed into the slit 100. In the present practical embodiment, as shown in FIG. 7, the effective radius r of the valve body 94 is the radius of the portion of the valve body 94 that is located on the radially inner side of the step 86 of the outer needle hub 74. Thus, in the present practical embodiment, the effective radius r of the valve body 94 is the same size as the radius of the straight part 84 in the lumen 76 of the outer needle hub 74.

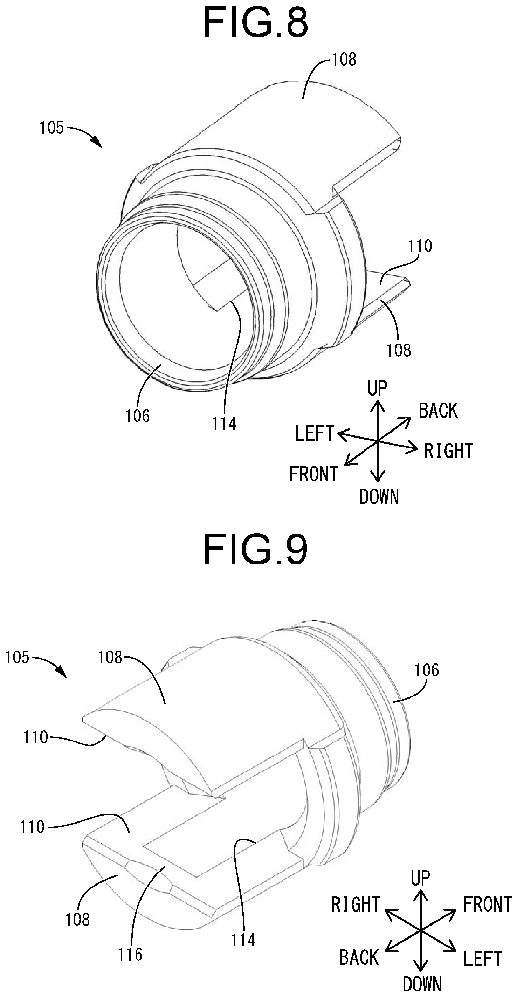

The valve body 94 is positioned in the outer needle hub 74 by the pusher guide 105. The pusher guide 105 holds the valve body 94 in position with respect to the outer needle hub 74, and guides the axial movement of the plunger 118, which is described below. As shown in FIGS. 8 and 9, the distal end portion of the pusher guide 105 has an approximately round tubular shape, and includes a distal end claw part 106 that is radially thin-walled and protrudes to the distal end side.

The proximal end portion of the pusher guide 105 includes a pair of guide pieces 108, 108 that are spaced apart from and face each other in the vertical direction. The pair of guide pieces 108, 108 are opposed to each other in the vertical direction and are approximately symmetrical in shape. Regarding each guide piece 108, the vertically outer surface has an arcuate cross section corresponding to the inner circumferential surface of the outer needle hub 74, while the vertically inner surface comprises a rotation restricting surface 110 whose curvature is smaller than that of the outer surface. The guide piece 108 is made thicker toward the circumferential center by the curvature of the vertically inner surface being smaller than that of the vertically outer surface.

At the distal end parts of the pair of guide pieces 108, 108, formed are respective groove-shaped recesses 114, 114 each opening on the inner circumferential surface and extending in the axial direction with an arcuate cross-section. The inner surface of the groove-shaped recess 114 has a curved concave shape in which the tubular inner circumferential surface of the pusher guide 105 on the distal end side with respect to the guide piece 108 extends to the proximal end side. The groove-shaped recess 114 does not reach the proximal end of the guide piece 108, and on the proximal end side with respect to the groove-shaped recess 114 in the guide piece 108, a retaining protruding part 116 protrudes inward in the direction of opposition of the pair of guide pieces 108, 108.

The pusher guide 105 is fixed to the outer needle hub 74 by the outer circumferential surface of the portion excluding the distal end claw part 106 being fitted against the inner circumferential surface of the outer needle hub 74.

Regarding the pusher guide 105, the distal end claw part 106 is inserted into the radial inside of the support tube part 98 of the valve body 94, and the support tube part 98 is radially clasped between the outer needle hub 74 and the pusher guide 105. The distal end portion of the distal end claw part 106 of the pusher guide 105 is inserted into the locking groove 102 of the valve body 94 and is axially butted against the valve body part 96 at the bottom surface of the locking groove 102, so as to be axially locked to the bottom surface of the locking groove 102. Furthermore, the outer peripheral end of the distal end surface of the valve body part 96 is axially overlapped on the step 86 of the outer needle hub 74, and the proximal end surface of the support tube part 98 is axially overlapped on the pusher guide 105. By so doing, the valve body 94 is positioned with respect to the outer needle hub 74. In the present practical embodiment, the outer diameter of the locking groove 102 (the inner diameter of the support tube part 98) is smaller than the effective diameter on the front surface side of the valve body 94 (the inner diameter of the step 86). The distal end surface of the distal end claw part 106 of the pusher guide 105 is in contact with the bottom surface of the locking groove 102 in the radially middle portion (away from the outer peripheral end). Thus, the step 86 and the distal end surface of the pusher guide 105, which are respectively in contact with the front and back sides of the valve body 94, are mutually shifted in the axial direction, and are not directly opposed to each other.

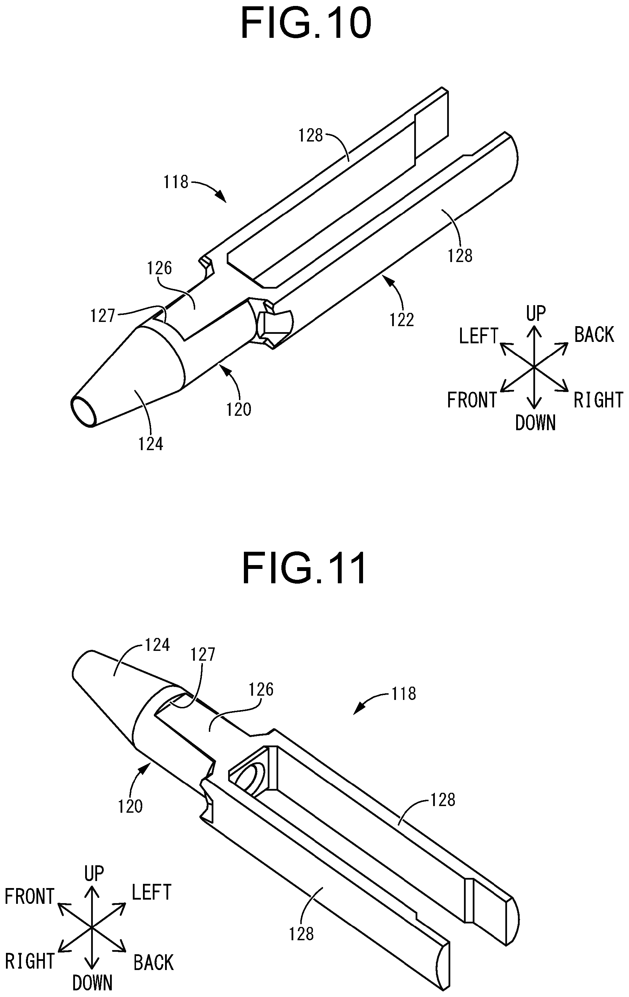

A plunger 118 is housed and disposed on the proximal end side of the valve body 94 within the lumen 76 of the outer needle hub 74. The plunger 118 pushes open the slit 100 of the valve body 94 to switch the valve body 94 to the communicating state by being pushed to the distal end side. As shown in FIGS. 10 to 13, the plunger 118 includes a tubular part 120 constituting its distal end portion, and a leg-shaped part 122 constituting its proximal end portion.

The tubular part 120 has a small-diameter, approximately round tubular shape, and is penetrated by a circular lumen in the axial direction. The inner diameter dimension of the tubular part 120 is approximately constant in the axial direction. The distal end part of the tubular part 120 comprises a tapered part 124 whose outer diameter dimension decreases toward the distal end.

In the tubular part 120, on the proximal end side with respect to the tapered part 124, the outer circumferential surface extends straightly without sloping with respect to the axial direction, and a plane part 126, which spreads approximately orthogonal to the vertical direction, is provided on each of the upper and lower side portions of the outer circumferential surface. The outer shape of the tubular part 120 is approximately a perfect circle on the distal end side with respect to the plane part 126, and is a non-perfect circle (a flat shape) on the proximal end portion where the plane part 126 is formed, in which the vertical dimension is smaller than the horizontal dimension.

With the plane part 126 being provided from the proximal end to the distal end of the tubular part 120 halfway in the axial direction, a restricting step 127 is formed on the distal end side of the plane part 126 on the outer circumferential surface of the tubular part 120. A pair of the restricting steps 127 are separately formed on the upper and lower sides due to the difference in outer diameter between the portion forming the plane part 126 and the distal end side with respect to the plane part 126, and extend in the direction intersecting the axial direction. The restricting step 127 in the present practical embodiment is a flat surface that extends in an approximately axis-perpendicular direction.

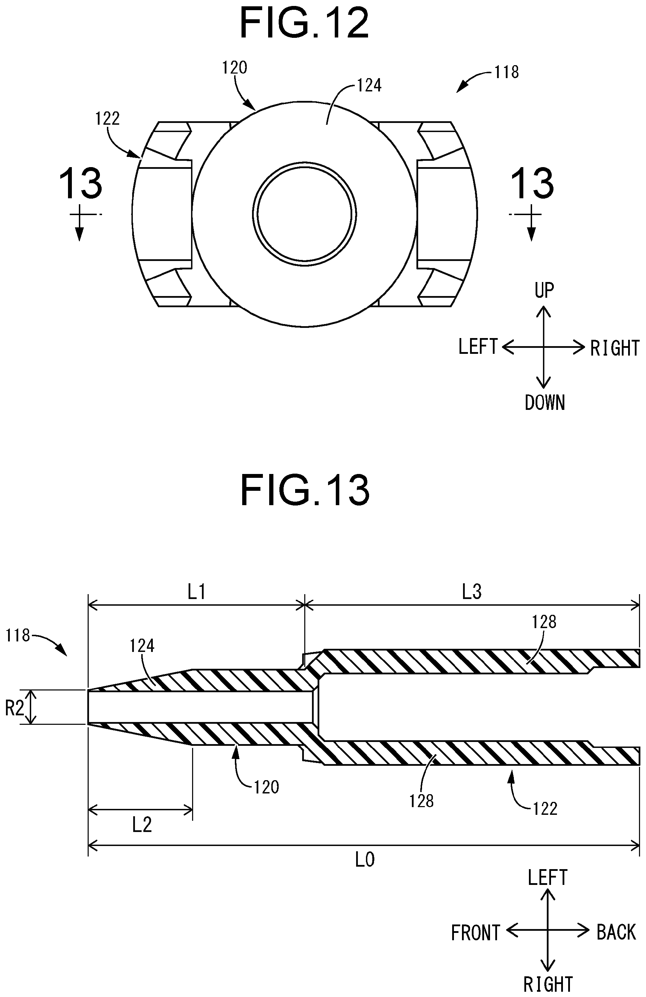

It is desirable that the entire length L0 of the plunger 118 (see FIG. 13) be at least three times as large as the axial length L2 of the tapered part 124 in the tubular part 120 of the plunger 118, and is suitably at least four times, more suitably at least five times. The entire length L0 of the plunger 118 is suitably ten times or less as large as the axial length L2 of the tapered part 124 in the tubular part 120 of the plunger 118, and more suitably seven times or less. Thus, the ratio of the axial length of the tapered part 124 in the plunger 118 is sufficiently large, and the axial length of the tapered part 124 can be reliably obtained while preventing the plunger 118 from being excessively elongated.

The axial length L2 of the tapered part 124 in the plunger 118 is suitably at least 1/4 of the axial length L1 of the tubular part 120, and more suitably at least 1/3. The axial length L2 of the tapered part 124 is suitably 3/4 or less as large as the axial length L1 of the tubular part 120. Thus, the ratio of the axial length of the tapered part 124 in the tubular part 120 is sufficiently large, and the axial length of the tapered part 124 can be reliably obtained while preventing the tubular part 120 from being excessively elongated.

It is desirable that the axial length L2 of the tapered part 124 be at least 2.5 mm, and is more suitably at least 3.0 mm. Besides, it is desirable that the axial length L2 of the tapered part 124 be 5 mm or less, and is more suitably 4 mm or less.

It is desirable that the axial length L2 of the tapered part 124 be at least twice as large as the outer diameter R2 of the distal end part of the tapered part 124 in the plunger 118, and suitably at least 2.5 times. Besides, it is desirable that the axial length L2 of the tapered part 124 be 4 times or less as large as the outer diameter R2 of the distal end part of the tapered part 124 in the plunger 118, and suitably 3.5 times or less. This prevents the tapered part 124 from being excessively elongated, and hence prevents the plunger 118 from being excessively elongated.

The leg-shaped part 122 includes a plurality of legs 128 extending toward the proximal end side. The leg-shaped part 122 of the present practical embodiment comprises a pair of the legs 128, 128 that are opposed to each other in the left-right direction. The leg 128 of the present practical embodiment has an approximately rectangular plate shape, and the left and right outer surfaces are arcuate curved surfaces in the circumferential direction corresponding to the inner circumferential surface of the outer needle hub 74. The pair of legs 128, 128 protrude outward to the left and right sides with respect to the tubular part 120. The axial length L3 of the leg 128 is longer than the axial length L1 of the tubular part 120. In this way, the axial length L1 of the tubular part 120 is relatively shortened, thereby inhibiting the plunger 118 and hence the outer needle hub 74 that houses the plunger 118 from being elongated.

The plunger 118 of the above construction is housed in the lumen 76 of the outer needle hub 74, as shown in FIGS. 2 and 3. The plunger 118 is arranged on the proximal end side of the valve body part 96 of the valve body 94, and the distal end portion of the tubular part 120 is inserted into the pusher guide 105. The pair of guide pieces 108, 108 of the pusher guide 105 extend to the proximal end side with respect to the tapered part 124 of the plunger 118, and the retaining protruding parts 116, 116 at the proximal end part of the guide pieces 108, 108 are located on the plane parts 126, 126 of the plunger 118. The retaining protruding parts 116, 116 of the pusher guide 105 overlap with the upper and lower pairs of restricting steps 127, 127 of the plunger 118 in a projected view in the axial direction. By the retaining protruding parts 116, 116 and the restricting steps 127, 127 being locked, a displacement restricting structure that limits the amount of displacement of the plunger 118 to the proximal end side is constituted. The restricting steps 127, 127 are provided on the tubular part 120 of the plunger 118, and are spaced apart to the distal end side from the leg-shaped part 122 of the plunger 118 housing the protector 30. Therefore, for example, the retaining protruding parts 116, 116 of the pusher guide 105, which are locked to the restricting steps 127, 127, can be prevented from interfering with the protector 30. With the plunger 118 axially positioned by the retaining protruding parts 116, 116, the distal end of the plunger 118 is close to or in contact with the thick convex part 103 of the valve body 94.

Besides, the restricting steps 127, 127 are provided in the tubular part 120 of the plunger 118 and are locked with the retaining protruding parts 116, 116 of the pusher guide 105. Thus, there is no need to provide a surface on the outer needle hub 74 side that is axially locked with the restricting steps 127, 127 directly on the inner surface of the outer needle hub 74. Therefore, there is no need to set an undercut shape on the inner surface of the outer needle hub 74 or to constitute the outer needle hub 74 with multiple parts. This achieves good formability and shape stability of the outer needle hub 74 with a small number of parts to enable stable mass production of the product.

The plunger 118 is entirely located and housed within the lumen 76 of the outer needle hub 74, with its proximal end located on the distal end side with respect to the proximal end of the outer needle hub 74. It is desirable that the length L2 (see FIG. 13) of the tapered part 124 in the plunger 118 be longer than the axial distance d (see FIG. 3) from the proximal end of the outer needle hub 74 to the proximal end of the plunger 118 (the proximal end of the leg 128), and is more suitably 1.4 times as large as the axial distance d. Besides, it is desirable that the length L2 of the tapered part 124 be 2.5 times or less as large as the axial distance d, and is more suitably twice or less as large as the axial distance d, which prevents the tapered part 124 from being excessively elongated. In the present practical embodiment, the plunger 118 is in a mounted state in which the distal end surface of the plunger 118 is in contact with the back surface of the valve body 94. Thus, the total value of the axial length L0 of the plunger 118 and the axial distance d from the proximal end of the plunger 118 to the opening of the outer needle hub 74 is the effective axial depth on the back surface side of the valve body 94 that ranges from the back surface to the opening end of the valve body 94 in the outer needle hub 74.

The axial length L2 of the tapered part 124 (see FIG. 13) is suitably larger than the effective radius r of the valve body 94 (see FIG. 7) attached to the outer needle hub 74, and more suitably at least twice as large as the effective radius r of the valve body 94. Besides, the axial length L2 of the tapered part 124 is suitably four times or less as large as the effective radius r of the valve body 94, and more suitably three times or less as large as the effective radius r of the valve body 94, which prevents the tapered part 124, with which the valve body 94 comes into contact after deformation, from being excessively elongated.

The outer diameter R2 of the distal end part of the tapered part 124 (see FIG. 13) is suitably smaller than twice the effective radius r of the valve body 94 (see FIG. 7), and more suitably equal to or less than the effective radius r of the valve body 94. The outer diameter R2 of the tapered part 124 is suitably at least 1/3 of the effective radius r of the valve body 94, and more suitably at least 1/2 of the effective radius r of the valve body 94. Thus, the distal end part of the tapered part 124, which is pushed into the deformable region of the valve body part 96 of the valve body 94, is sufficiently large in diameter with respect to the said deformable region, thereby making it possible to effectively open the valve body 94 as well as to sufficiently obtain the opening area of the valve body 94 in the communicating state. The inner diameter of the straight part 84 in the outer needle hub 74 is larger than the outer diameter of the proximal end part of the tapered part 124, which is the maximum outer diameter dimension of the tapered part 124.

The width dimension W of the outer circumferential lightening part 104 in the locking groove 102 of the valve body 94 is suitably smaller than the outer diameter R2 of the distal end part of the plunger 118, and more suitably 2/3 or less than the outer diameter R2 of the distal end part of the plunger 118. Besides, the width dimension W of the outer circumferential lightening part 104 is suitably at least 1/4 of the outer diameter R2 of the distal end part of the plunger 118, and more suitably at least 1/3 of the outer diameter R2 of the distal end part of the plunger 118. This makes it possible to provide the thin-walled portion of the valve body 94 formed by the outer circumferential lightening part 104 with a sufficient width.

The outer needle unit 14, which includes the outer needle hub 74 housing the valve mechanism comprising the valve body 94, the pusher guide 105, and the plunger 118, is combined with the inner needle unit 12 to form the valved indwelling needle assembly 10. That is, the inner needle 16 is inserted through the outer needle 72 and the lumen 76 of the outer needle 72 from the proximal end side, and the connecting part 26 of the inner needle hub 18 is fitted into the proximal end opening portion of the outer needle hub 74, so that the inner needle unit 12 and the outer needle unit 14 are mutually combined to form the valved indwelling needle assembly 10.

In the valved indwelling needle assembly 10, the inner needle 16 penetrates the slit 100 of the valve body 94. The inner needle 16 is also inserted through the tubular part 120 of the plunger 118. The distal end portion of the inner needle 16 protrudes to the distal end side with respect to the outer needle 72, and the needle tip 20 is exposed without being covered by the outer needle 72.

The protector 30 attached to the inner needle 16 is housed in the lumen 76 of the outer needle hub 74 on the proximal end side with respect to the plunger 118. The protector 30 is arranged between the pair of legs 128, 128 of the plunger 118, with the first and second side plates 34, 36 and the first and second needle-tip protection parts 44, 58 exposed to the upper and lower sides between the pair of legs 128, 128. The pair of legs 128, 128 of the plunger 118 extend on the left and right sides of the protector 30 toward the proximal end side, with the proximal end reaching near the bottom part 32 of the protector 30. In this way, the protector 30 is arranged between the opposed legs 128, 128 of the plunger 118, and the legs 128, 128 are located on the left and right outer sides of the protector 30. This makes it possible to reliably obtain a space for disposing the protector 30 as well as to allow the plunger 118 to extend out to near the proximal end opening of the outer needle hub 74. Regarding the plunger 118 of the present practical embodiment, the axial length L3 of the leg-shaped part 122 comprising the pair of legs 128, 128 is longer than the axial length L1 of the tubular part 120. This reliably obtains the sufficient space for disposing the protector 30.

The first and second needle-tip protection parts 44, 58 of the protector 30 are arranged in the protector housing part 80 of the lumen 76 of the outer needle hub 74. The protector housing part 80 has a concave cross-section and a large inner diameter, so as to be able to house the first and second needle-tip protection parts 44, 58, which protrude to the upper and lower outer sides with respect to the first and second side plates 34, 36. The first and second needle-tip protection parts 44, 58 of the protector 30 are axially apart from the tubular part 120 of the protector 30 to the proximal end side.

In the inner needle hub 18, a portion in the circumferential direction of the connecting part 26 protrudes more significantly to the distal end side and is inserted below the second side plate 36 of the protector 30. This limits the rotation of the protector 30 relative to the inner needle hub 18, and keeps the protector 30 in a proper orientation around the needle axis. The inner needle hub 18 and the outer needle hub 74 are circumferentially positioned with respect to each other using, for example, the screw part 78. Hence, the rotation of the protector 30 relative to the outer needle hub 74 is also restricted via the inner needle hub 18, and the protector 30 is housed in the lumen 76 of the outer needle hub 74 in a proper orientation.

Regarding the valved indwelling needle assembly 10, for example, the inner needle 16 and the outer needle 72 puncture a blood vessel of a patient or the like. Blood flows into the lumen of the inner needle hub 18 through the lumen of the inner needle 16 when puncturing a blood vessel. Thus, for example, by making the inner needle hub 18 transparent or translucent so that the interior can be seen, the proper puncture of the inner needle 16 into the blood vessel can be confirmed by the inflow of the blood into the inner needle hub 18. Besides, a filter of the filter cap 28 attached to the inner needle hub 18 prevents the blood that has flowed into the lumen of the inner needle hub 18 from leaking out of the opening on the proximal end side.

After puncturing the blood vessel with the inner needle 16 and the outer needle 72, the inner needle 16 will be withdrawn from the outer needle 72 to the proximal end side by pulling the inner needle unit 12 toward the proximal end side while holding the outer needle unit 14 in the puncture position.

When the inner needle 16 is pulled out of the outer needle 72 to the proximal end side, the protector 30, which is housed in the protector housing part 80 of the outer needle hub 74, is restricted in its movement to the proximal end side by the first and second needle-tip protection parts 44, 58 getting caught on the inner surface of the protector housing part 80. By so doing, due to withdrawal of the inner needle 16, the protector 30 moves toward the needle tip 20 side while sliding against the outer circumferential surface of the inner needle 16. When the needle tip 20 of the inner needle 16 moves to the proximal end side with respect to the first and second needle-tip protection parts 44, 58, the first and second needle-tip protection parts 44, 58 are allowed to approach each other, and the first and second needle-tip protection parts 44, 58 approach each other based on the elasticity of the first and second side plates 34, 36, or the like. As a result, the needle tip 20 of the inner needle 16 is covered at the distal end by the first and second needle-tip protection parts 44, 58, and the needle tip 20 is protected by the first and second needle-tip protection parts 44, 58. In such a needle tip protection state, re-exposure of the needle tip 20 to the distal end side is prevented. In the needle tip protection state, the first and second needle-tip protection parts 44, 58 and the protector housing part 80 of the inner needle hub 18 are released from being locked, and the axial movement of the protector 30 with respect to the outer needle hub 74 is allowed.

By further pulling out the inner needle 16 from the needle tip protection state, the detent part 24 of the inner needle 16 is locked to the bottom part 32 of the protector 30. By even further pulling out the inner needle 16 with the detent part 24 and the bottom part 32 locked, the protector 30 moves with the inner needle 16 to the proximal end side relative to the outer needle hub 74, and is withdrawn from the outer needle hub 74 to the proximal end side. By so doing, the inner needle unit 12, with the needle tip 20 of the inner needle 16 protected by the protector 30, is separated from the outer needle unit 14, and the outer needle unit 14 is indwelled in a state of puncturing the blood vessel. Thus, in the present practical embodiment, the outer needle unit 14 serves as a valved indwelling needle.

In the indwelled outer needle unit 14, due to withdrawal of the inner needle 16, the slit 100 of the valve body 94 is closed, so that the lumen 76 of the outer needle hub 74 is blocked by the valve body 94. This makes it possible to avoid the problem of blood in the blood vessel leaking to the outside through the lumen 76 of the outer needle hub 74.

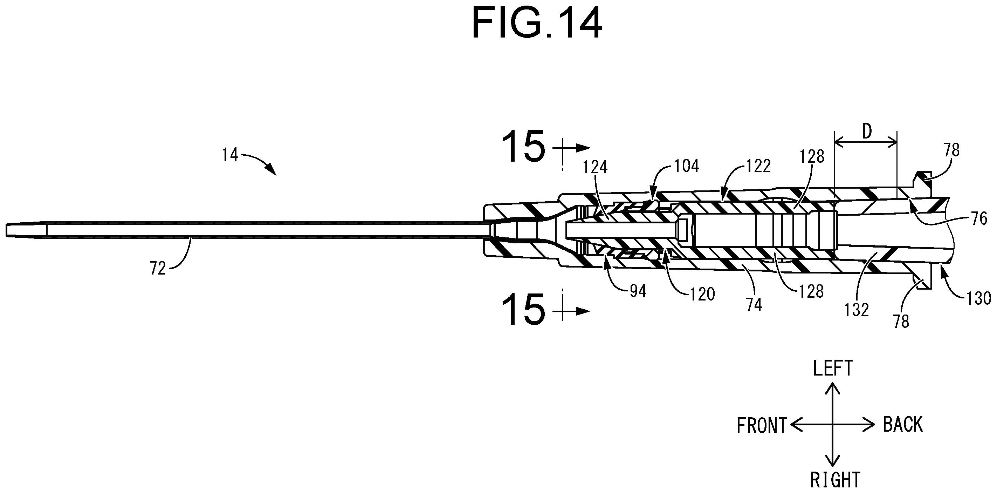

A connector 130 is connected to the indwelled outer needle unit 14, as shown in FIG. 14. The connector 130 is, for example, a syringe tip or an infusion line connector, and includes a male luer 132 that is fitted into the proximal end portion of the outer needle hub 74. By the male luer 132 being fitted into the proximal end portion of the outer needle hub 74, the connector 130 is connected to the outer needle unit 14. The outer circumferential surfaces of the male luer 132 and the inner circumferential surface of the outer needle hub 74, to which respective luer tapers are set, are fitted to each other in mutually close contact, so that the male luer 132 is axially positioned with respect to the outer needle hub 74. The connector 130 may be provided with a lock part (not shown) that is screwed onto the screw part 78 of the outer needle hub 74 to maintain the connected state between the connector 130 and the outer needle hub 74. FIG. 14 shows the male luer 132 made of a thin, soft material and pushed in strongly using torque from tightening the lock part (not shown), and the axial length of the portion of the male luer 132 that is inserted into the outer needle hub 74 is 6.5 mm.

Generally, the male luer 132 and the outer needle hub 74 are connected using the torque of the lock part. When using the torque of the lock part for connecting the male luer 132 and the outer needle hub 74, the user tends to tighten the lock part strongly, and the plunger 118 may be pushed in longer to the distal end side with respect to the valve body 94. However, when a male luer made of thick and hard material was used, the plunger moved little even if the torque of the lock part was used, and it was demonstrated that with the conventional plunger, which is returned to its initial position by the reaction force of the valve body 94, the plunger did not obtain enough reaction force to return sufficiently even if the male luer is removed. In the present practical embodiment of the outer needle unit 14, the axial length of the plunger 118 is sufficiently long, with the axial length of the tapered part 124 in the plunger 118 being larger than the effective radius r of the valve body 94, while the entire axial length of the plunger 118 being at least three times as large as the axial length of the tapered part 124. With this configuration, it was demonstrated that when inserting the male luer 132 into the outer needle hub 74 as described below, even if the insertion of the male luer 132 was shallow, the plunger 118, which was extended to a location close to the proximal end opening of the outer needle hub 74, sufficiently moved to the distal end side, and a large contact reaction force (elastic force) of the valve body 94 acting on the tapered part 124 of the plunger 118 was readily obtained, thereby improving the stability of the blocking function of the valve body 94.

Meanwhile, an indwelling needle for infusion is required to be small because it is indwelled in various places. In a conventional plunger, which is returned to its initial position by the reaction force of the valve body 94, the distal end outer diameter of the tapered part is large and the axial length of the tapered part is short relative to the size of the valve body 94. Accordingly, it was found that the range of compatibility of the connector with the returnability of the plunger and the blocking functionality of the valve body 94 was narrow, and the returning force (the reaction force of the valve body 94) that pushed back the plunger, which was pushed in by the male luer 132 inserted into the outer needle hub 74, to the proximal end side was difficult to stably obtain due to variations in manufacturing error or hardness, differences in the amount of pushing by the user, and other factors. For example, it was demonstrated that when the plunger is pushed in excessively, the valve body 94 overrode beyond the tapered part of the plunger, and the plunger did not return to the proximal end side even after the male luer 132 is removed from the outer needle hub 74. On the other hand, regarding the outer needle unit 14 in the present practical embodiment, the axial length of the tapered part 124 of the plunger 118 is at least twice as large as the outer diameter of the distal end part, while the outer diameter of the distal end part of the tapered part 124 is equal to or less than the effective radius of the valve body 94, so that the outer diameter the distal end part of the tapered part 124 is set small relative to the valve body 94. This makes it possible to reliably obtain a large inclination angle of the tapered part 124 while setting the axial length of the tapered part 124 long. Therefore, variations in the amount of pushing of the plunger 118 by the male luer 132 are allowed in a wider range, and the returning force acting on the tapered part 124 of the plunger 118 is stabilized, so that stabilization of the blocking function of the valve body 94 can be expected. Besides, the axial length of the tapered part 124 of the plunger 118 is at least twice as large as the effective radius of the valve body 94, and the axial length L2 of the tapered part 124 of the plunger 118 is set to be longer than the effective radius r of the valve body 94, thereby avoiding increased size of the outer needle hub 74, which is suitable for infusion.

When the outer needle unit 14 and the connector 130 are connected, the distal end of the connector 130 comes into contact with the pair of legs 128, 128 of the plunger 118 from the proximal end side, and the plunger 118 is pushed to the distal end side by the connector 130. As shown in FIG. 14, the plunger 118 moves to the distal end side, and the tapered part 124 of the plunger 118 pushes open the slit 100 of the valve body 94 so as to be inserted through the slit 100. By so doing, the blockage of the lumen 76 of the outer needle hub 74 by the valve body 94 is released, and the blood vessel and the connector 130 are interconnected via the lumen 76 of the outer needle unit 14. The outer needle unit 14 to which the connector 130 is connected can then be used to perform medication, infusion, or the like into the blood vessel, or to perform blood collection or the like from the blood vessel. The distal end outer peripheral part of the valve body 94 is in contact with the step 86 in the outer needle hub 74, and the tapered part 124 of the plunger 118 is located on the radially inner side of the step 86. From the viewpoint of stabilizing the returnability of the plunger 118, in the case where the axial length of the male luer 132 within the outer needle hub 74 (i.e., the insertion length of the male luer 132) is 6.5 mm, the tapered part 124 of the plunger 118 is preferably located on the radially inner side of the step 86. Also, in the case where the axial length of the male luer 132 within the outer needle hub 74 is 4.5 mm, the tapered part 124 is preferably located on the radially inner side of the step 86.

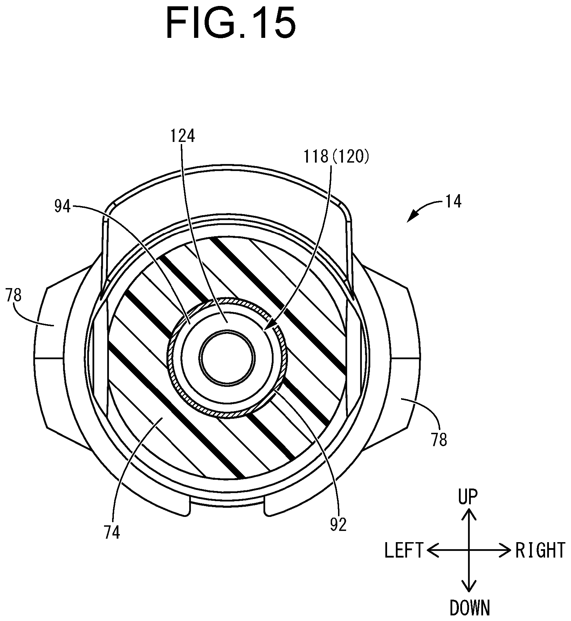

In the communicating state of the valve body 94, the tubular part 120 of the plunger 118 penetrates the slit 100 of the valve body 94, and as also shown in FIG. 15, the distal end opening of the plunger 118 is open without being covered by the valve body 94 when viewed in the axial direction from the distal end side.

In the present practical embodiment, the axial distance d from the proximal end of the outer needle hub 74 to the proximal end of the plunger 118 is shorter than the axial length L2 of the tapered part 124 of the plunger 118, so that the proximal end of the plunger 118 is sufficiently close to the proximal end opening of the outer needle hub 74. Hence, by inserting the male luer 132 of the connector 130 into the proximal end opening portion of the outer needle hub 74, the plunger 118 is pushed sufficiently to the distal end side, and the valve body 94 is stably switched into the communicating state by the plunger 118.

It is desirable that the amount of movement D of the plunger 118 to the distal end side due to connection of the connector 130 with the outer needle hub 74 be equal to or less than the axial length L2 of the tapered part 124 in the plunger 118. The amount of movement D of the plunger 118 to the distal end side is suitably at least 50%, and more suitably at least 80%, of the axial length L2 of the tapered part 124. By setting the amount of movement D of the plunger 118 to the distal end side in this way, the connection of the connector 130 allows the tapered part 124 of the plunger 118 to be stably inserted through the slit 100 of the valve body 94, thereby stabilizing the communication operation of the valve body 94.

In the present practical embodiment, the plunger 118 is in a mounted state in which the distal end surface of the plunger 118 is in contact with the back surface of the valve body 94. Thus, when connecting the connector 130 with the outer needle hub 74, the difference between the insertion length of the connector 130 into the outer needle hub 74 and the axial distance d from the proximal end of the plunger 118 to the opening part of the outer needle hub 74 is equal to the axially forward pushed length of the plunger 118 by the connector 130, and hence the amount of forward movement D of the plunger 118. Therefore, in the present practical embodiment, the connection of the connector 130 with the outer needle hub 74 causes the tubular part 120 on the distal end side of the plunger 118 to be inserted into the valve body 94 by such an amount of movement D to push open the slit 100.

Regarding the plunger 118, the axial length L3 of the leg-shaped part 122 comprising the pair of legs 128, 128 is longer than the axial length L1 of the tubular part 120. By providing such a long leg-shaped part 122, the guiding action of the left and right outer surfaces of the leg-shaped part 122 being guided by the inner circumferential surface of the outer needle hub 74 can be advantageously obtained, thereby achieving stabilization in axial movement of the plunger 118. Moreover, the fact that the entire axial length L0 of the plunger 118 is at least three times as large as the axial length L2 of the tapered part 124 also achieves stabilization in movement of the plunger 118 due to the guiding action.

The distal end of the plunger 118 comes into contact with the proximal end surface of the thick convex part 103 of the valve body 94, and is inserted into the slit 100 while pushing the thick convex part 103 to the distal end side and deforming the valve body 94. The locking groove 102 provided on the radially outer side with respect to the valve body 94 extends further radially inward than the distal end claw part 106 of the pusher guide 105, and the radially inner portion with respect to the distal end claw part 106 comprises the outer circumferential lightening part 104. The portion forming the outer circumferential lightening part 104 in the valve body 94 is not restrained by the pusher guide 105. This makes it easy to allow deformation of the valve body 94 at the thin-walled portion forming the outer circumferential lightening part 104, thereby enabling smooth switching of the valve body 94 to the communicating state.

In the present practical embodiment, the width dimension W of the outer circumferential lightening part 104 is 2/3 or less than the outer diameter R2 of the distal end part of the tapered part 124 of the plunger 118. With this configuration, deformation of the valve body 94 due to the plunger 118 being pushed in is stably allowed by the thin-walled portion forming the outer circumferential lightening part 104, and the thin-walled portion in the valve body 94 is prevented from becoming excessively large to reliably obtain the shape recovering force based on the elasticity of the valve body 94.

With the plunger 118 pushed in the valve body 94, the plunger 118 moves to the distal end side to a location where its distal end part enters the radial inside of the caulking pin 92. The distal end part of the plunger 118 is inserted into the portion where the caulking pin 92 is attached to the constricted part 90 of the lumen 76 of the outer needle hub 74, and does not reach the distal end portion of the caulking pin 92, which has a smaller diameter.

In this communicating state of the valve body 94, the valve body part 96 of the valve body 94, which has been deformed by its slit 100 being pushed open, is pressed against the outer circumferential surface of the tapered part 124 in the plunger 118. A force based on the elasticity of the valve body 94 is exerted on the outer circumferential surface of the tapered part 124 on which the deformed valve body 94 is overlapped. Since the tapered part 124 has a tapered shape, the force exerted on the outer circumferential surface of the tapered part 124 urges the plunger 118 inward in the radial direction and toward the proximal end side. However, with the connector 130 connected to the outer needle hub 74, the positioning force acting between the outer needle hub 74 and the connector 130 is greater than the force pushing the plunger 118 to the proximal end side, and the plunger 118 is held in the location where the plunger 118 penetrates the slit 100 of the valve body 94.

A straight part 84 is provided in the lumen 76 of the outer needle hub 74 between the valve body 94 and the caulking pin 92, and the axial length L4 of the straight part 84 is larger than the effective radius r of the valve body 94. Hence, the valve body 94, which has been deformed to the distal end side by the plunger 118 pushing in, is less likely to enter the radial inside of the caulking pin 92 fitted in the constricted part 90, and the opening operation of the valve body 94 is achieved smoothly. Besides, it is also possible to prevent the movement of the plunger 118 from being obstructed by the valve body 94 that is clasped between the plunger 118 and the caulking pin 92.

Meanwhile, when the connector 130 is removed from the outer needle unit 14, blood leaks to the outside if the valve body 94 remains in the communicating state. Therefore, the outer needle unit 14 is configured such that the valve body 94 switches from the communicating state to the blocking state when the connector 130 is removed.

That is, when the connector 130 is removed from the outer needle unit 14, the force due to the connector 130 pushing the plunger 118 to the distal end side is released. In the communicating state of the valve body 94, the valve body 94 in the deformed state is pressed against the outer circumferential surface of the tapered part 124 in the plunger 118, and the axial component of the elastic force of the valve body 94 acts as a returning force toward the proximal end side against the plunger 118. Accordingly, the plunger 118 is pushed back to the proximal end side in association with the valve body 94 recovering its shape to the blocking state, so that the plunger 118 returns to the location before being pushed by the connector 130, and the slit 100 of the valve body 94 closes to block the lumen 76 of the outer needle hub 74. In this way, in the outer needle unit 14, pushing back of the plunger 118 to the proximal end side is realized by using the elastic force of the valve body 94 without requiring separate urging means. Hence, the number of parts of the outer needle unit 14 having an internal valve mechanism can be reduced.

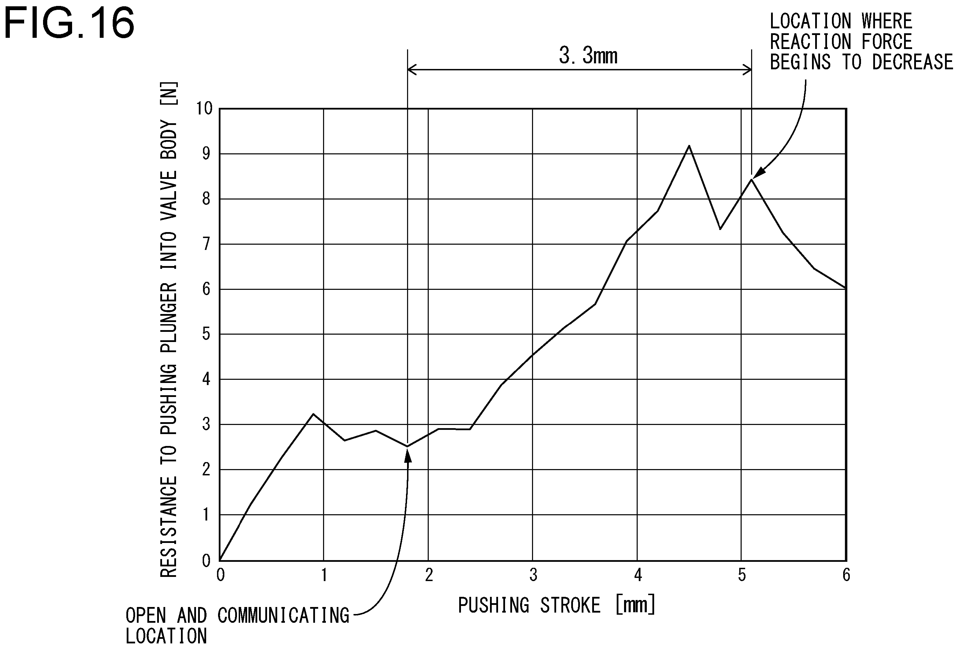

FIG. 16 shows a graph of the measurement results of resistance to pushing (returning force) due to the elasticity of the valve body 94 against the pushing stroke of the plunger 118 to the distal end side. In this graph, if the plunger 118 is located in a stroke region where the resistance to pushing increases or is maintained in response to an increase in the pushing stroke, the plunger 118 is effectively pushed back to the proximal end side by the elasticity of the valve body 94 when the connector 130 is disconnected. In the graph in FIG. 16, the open and communicating location of the plunger 118 refers to the location where the plunger 118 penetrates the valve body 94 and the entire lumen of the plunger 118 is visible and completely open without being covered by the valve body 94 when viewed in the axial direction from the distal end side. Besides, in the graph in FIG. 16, the location where the reaction force of the plunger 118 begins to decrease refers to the location where the reaction force to the proximal end side (the resistance to pushing) acting on the plunger 118 due to the elasticity of the valve body 94 begins to decrease without turning to increase later in response to the increase in the pushing stroke.

The graph in FIG. 16 shows that regarding the outer needle unit 14 in the present practical embodiment, the plunger 118 reaches the open and communicating location when the pushing stroke of the plunger 118 becomes 1.8 mm, and the plunger 118 reaches the location where the reaction force begins to decrease when the pushing stroke of the plunger 118 becomes 5.1 mm. That is, at least in the pushing region until the pushing stroke of the plunger 118 reaches 5.1 mm, the resistance to pushing that acts on the plunger 118 is kept greater than the reaction force that acts on the plunger 118 at the open and communicating location. In other words, regarding the outer needle unit 14 in the present practical embodiment, in the pushing region of the plunger 118 in which the plunger 118 extends 3.3 mm further from the open and communicating location, the resistance to pushing the plunger 118 into the valve body 94 is kept greater than the resistance to pushing at the open and communicating location.

Therefore, if the pushing stroke of the plunger 118 is 1.8 mm or greater, the plunger 118 can stably switch the valve body 94 to the communicating state by pushing of the plunger 118. Besides, if the pushing stroke of the plunger 118 is 5.1 mm or less, the return action on the plunger 118 to the proximal end side due to the elasticity of the valve body 94 is stably achieved when the connector 130 is disconnected, and the valve body 94 is switched to the blocking state.

The plunger 118 used in the measurement that obtained the results shown in FIG. 16 is configured such that the length L2 of the tapered part 124 is 3.4 mm, the minimum outer diameter (the distal end outer diameter) R2 of the tapered part 124 is 1.0 mm, and the maximum outer diameter (the proximal end outer diameter) of the tapered part 124 is 2.45 mm.

In the outer needle unit 14 based on the results of FIG. 16, the axial length of the tapered part 124 is at least 3 mm. Furthermore, in relation to the resistance to pushing the plunger 118 into the valve body 94 and the location of the plunger 118, in the pushing region of the plunger 118 that extends 3 mm further from the open and communicating location where the opening of the valve body 94 opened by the plunger 118 being pushed into the valve body 94 reaches the size equal to the inner hole diameter of the plunger 118, the value of resistance to pushing the plunger 118 into the valve body 94 is kept greater than that at the open and communicating location. In the present practical embodiment, the pushing region in which the plunger 118 that has reached the open and communicating location can move further to the distal end side is provided, and the tapered part 124 has a taper length that allows the elastic returning force of the valve body 94 to act sufficiently on the tapered part 124 even in the pushing region. With these configurations, compared to plungers that cause conventional return action, the plunger 118 of present practical embodiment has a wider range of compatibility with the insertion length of the male luer 132. Therefore, even if the insertion length of the male luer 132 varies due to variations in manufacturing error or hardness, differences in the pushing force by the user, and other factors, it is possible to stably obtain the returning force to the proximal end side for the plunger 118 that is pushed into the valve body. It is also possible to achieve the outer needle unit 14 that causes a stable return action even when the shape and dimensions of each component such as the plunger 118 are appropriately changed from the illustrated shape and dimensions.

Conventionally known male luer have some variations in dimensions and hardness. Regarding a thin male luer, the insertion length into the outer needle hub 74 is longer than that of a thick male luer. In addition, a male luer made of a soft material is inserted into the outer needle hub 74 while being deformed, resulting in a longer insertion length into the outer needle hub 74 compared to a male luer made of a hard material. Therefore, a thick and hard male luer and a thin and soft male luer were prepared, and inserted into the outer needle unit 14 of the present practical embodiment. Regarding the thick and hard male luer, the insertion length of the male luer into the outer needle hub 74 was 4.5 mm, the pushing stroke of the plunger 118 was 2.5 mm, and the resistance to pushing the plunger 118 into the slit 100 of the valve body 94 was at least 3 N. Regarding the thin and soft male luer, the insertion length of the male luer into the outer needle hub 74 was 6.5 mm, the pushing stroke of the plunger 118 was 4.5 mm, and the resistance to pushing the plunger 118 into the slit 100 of the valve body 94 was at least 3 N. In both cases, it was demonstrated that when the male luer was removed, the return action on the plunger 118 to the proximal end side was stably achieved, and the valve body 94 switched to the blocking state.

Accordingly, it was found that, in preferred practice, the resistance to pushing the plunger 118 into the slit 100 of the valve body 94 was at least 3 N, in both cases where the insertion length of the male luer into the outer needle hub 74 was 4.5 mm and where the insertion length of the male luer into the outer needle hub 74 was 6.5 mm. Note that stable return action on the plunger 118 to the proximal end side was confirmed even when the resistance to pushing the plunger 118 into the slit 100 of the valve body 94 was 2.5 N. Thus, the said resistance is preferably at least 2.5 N in both cases where the insertion length of the male luer into the outer needle hub 74 is 4.5 mm and where the insertion length of the male luer into the outer needle hub 74 is 6.5 mm.

A practical embodiment of the present invention has been described in detail above, but the present invention is not limited to those specific descriptions. For example, it is desirable for the protector 30 to have the structure in which the needle tip protectors 44, 58 are provided on the distal end side of the pair of side plates 34, 36 arranged so as to face each other, for efficient use of the space for disposition, etc. However, the protector 30 is not limited in its specific structure.

In the valved indwelling needle assembly according to the preferred embodiments other than the fourth and eighth preferred embodiments described above, the plunger is not necessarily limited to the structure including the leg-shaped parts, but may be entirely tubular in shape, for example.

### KEYS TO SYMBOLS

- 10: valved indwelling needle assembly (first practical embodiment)
- 12: inner needle unit
- 14: outer needle unit
- 16: inner needle
- 18: inner needle hub
- 20: needle tip
- 22: blade surface
- 24: detent part
- 26: connecting part
- 28: filter cap
- 30: protector
- 32: bottom part
- 34: first side plate
- 36: second side plate
- 38: needle insertion hole
- 40: needle guide surface
- 42: reinforcing tube part
- 44: first needle-tip protection part
- 46: first stepped part
- 48: first needle-tip cover part
- 50: first needle-tip stopper part
- 52: first lateral guarding piece
- 54: first lateral cover piece
- 56: first protrusion piece
- 58: second needle-tip protection part
- 60: second stepped part
- 62: second needle-tip cover part
- 64: second needle-tip stopper part
- 66: second lateral guarding piece
- 68: second lateral cover piece
- 70: second protrusion piece
- 72: outer needle
- 74: outer needle hub
- 76: lumen
- 78: screw part
- 80: protector housing part
- 82: valve housing part
- 84: straight part (deformation allowance part)
- 86: step
- 88: caulking part
- 90: constricted part
- 92: caulking pin
- 94: valve body
- 96: valve body part
- 98: support tube part
- 100: slit
- 102: locking groove
- 103: thick convex part
- 104: outer circumferential lightening part
- 105: pusher guide
- 106: distal end claw part
- 108: guide piece
- 110: rotation restricting surface
- 114: groove-shaped recess
- 116: retaining protruding part
- 118: plunger
- 120: tubular part
- 122: leg-shaped part
- 124: tapered part
- 126: plane part
- 127: restricting step
- 128: leg
- 130: connector
- 132: male luer
- L0: entire axial length of plunger
- L1: axial length of tubular part
- L2: axial length of tapered part
- L3: axial length of leg-shaped part
- L4: axial length of straight part
- R1: outside diameter of distal end claw part of pusher guide
- R2: outside diameter of distal end part of tapered part
- r: effective radius of valve body
- W: radial width dimension of outer circumferential lightening part
- d: axial distance from proximal end of outer needle hub to proximal end of plunger
- D: pushing stroke of plunger to distal end side

## Claims

1. A valved indwelling needle assembly comprising:
an inner needle including an inner needle hub, the inner needle being inserted through an outer needle and an outer needle hub;
a valve body opening and closing through elastic deformation; and
a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein
the plunger includes a tapered part on the distal end side,
an entire axial length of the plunger is at least three times as large as an axial length of the tapered part,
the plunger is entirely located within the outer needle hub, and
the axial length of the tapered part is larger than an effective radius of the valve body where free deformation is allowed on a distal end surface of the valve body.

2. The valved indwelling needle assembly according to claim 1, wherein
the axial length of the tapered part is at least 3 mm, and
in a pushing region of the plunger that extends 3 mm further from an open and communicating location where an opening of the valve body opened by the plunger being pushed into the valve body reaches a size equal to an inner hole diameter of the plunger, a value of resistance to pushing the plunger into the valve body is kept greater than that at the open and communicating location.

3. The valved indwelling needle assembly according to claim 1 or 2, wherein
the axial length of the tapered part is at least twice as large as an outer diameter of a distal end part of the tapered part, and
the outer diameter of the distal end part of the tapered part is equal to or less than the effective radius of the valve body where free deformation is allowed on the distal end surface of the valve body.

4. The valved indwelling needle assembly according to any one of claims 1-3, wherein
the plunger comprises a tubular part whose distal end side has a tapered shape, and a leg-shaped part including a plurality of legs extending toward the proximal end side,
the tubular part includes a restricting step that restricts a movement of the plunger to the proximal end side relative to the outer needle hub by contact with an outer needle hub side,
a protector is arranged in the leg-shaped part, the protector protecting a needle tip when the inner needle is withdrawn, and
an axial length of the leg-shaped part is longer than an axial length of the tubular part.

5. The valved indwelling needle assembly according to any one of claims 1-4, wherein
the lumen of the outer needle hub includes a deformation allowance part extending straightly from a distal end of the valve body toward the distal end side with a substantially constant diameter while having an inner diameter larger than a maximum diameter of the tapered part,
an axial length of the deformation allowance part is larger than the effective radius of the valve body, and
the axial length of the tapered part is longer than an axial distance from a proximal end of the outer needle hub to a proximal end of the plunger that is located on the distal end side with respect to the proximal end of the outer needle hub.

6. A valved indwelling needle assembly comprising:
an inner needle including an inner needle hub, the inner needle being inserted through an outer needle and an outer needle hub;
a valve body opening and closing through elastic deformation; and
a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein
the plunger includes a tapered part on the distal end side,
an axial length of the tapered part is at least 3 mm, and
in a pushing region of the plunger that extends 3 mm further from an open and communicating location where an opening of the valve body opened by the plunger being pushed into the valve body reaches a size equal to an inner hole diameter of the plunger, a value of resistance to pushing the plunger into the valve body is kept greater than that at the open and communicating location.

7. A valved indwelling needle assembly comprising:
an inner needle including an inner needle hub, the inner needle being inserted through an outer needle and an outer needle hub;
a valve body opening and closing through elastic deformation; and
a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein
the plunger includes a tapered part on the distal end side,
an axial length of the tapered part is at least twice as large as an outer diameter of a distal end part of the tapered part, while being at least twice as large as an effective radius of the valve body, and
the outer diameter of the distal end part of the tapered part is equal to or less than the effective radius of the valve body where free deformation is allowed on a distal end surface of the valve body.

8. A valved indwelling needle assembly comprising:
an inner needle including an inner needle hub, the inner needle being inserted through an outer needle and an outer needle hub;
a valve body opening and closing through elastic deformation; and
a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein
the plunger comprises a tubular part whose distal end side has a tapered shape, and a leg-shaped part including a plurality of legs extending toward the proximal end side,
the tubular part includes a restricting step that restricts a movement of the plunger to the proximal end side relative to the outer needle hub by contact with an outer needle hub side,
a protector is arranged in the leg-shaped part, the protector protecting a needle tip when the inner needle is withdrawn, and
an axial length of the leg-shaped part is longer than an axial length of the tubular part.

9. A valved indwelling needle assembly comprising:
an inner needle including an inner needle hub, the inner needle being inserted through an outer needle and an outer needle hub;
a valve body opening and closing through elastic deformation; and
a plunger arranged on a proximal end side with respect to the valve body and turning the valve body into a communicating state by being pushed to a distal end side while receiving a returning force toward the proximal end side due to a reaction force from the valve body in the communicating state of the valve body, the valve body and the plunger being housed within a lumen of the outer needle hub, wherein
the plunger includes a tapered part on the distal end side, and the lumen of the outer needle hub includes a deformation allowance part extending straightly from a distal end of the valve body toward the distal end side with a substantially constant diameter while having an inner diameter larger than a maximum diameter of the tapered part,
an axial length of the deformation allowance part is larger than an effective radius of the valve body, and
an axial length of the tapered part is longer than an axial distance from a proximal end of the outer needle hub to a proximal end of the plunger that is located on the distal end side with respect to the proximal end of the outer needle hub.
